(19) Europäisches Patentamt
European Patent Office
Office européen des brevets

(11) Veröffentlichungsnummer: **0 287 828**
**A1**

(12) # EUROPÄISCHE PATENTANMELDUNG

(21) Anmeldenummer: **88104690.8**

(22) Anmeldetag: **24.03.88**

(51) Int. Cl.4: **C07D 211/90 , C07D 413/04 , C07D 405/04**

(30) Priorität: **27.03.87 CH 1193/87**

(43) Veröffentlichungstag der Anmeldung: **26.10.88 Patentblatt 88/43**

(84) Benannte Vertragsstaaten: **ES GR**

(71) Anmelder: **Byk Gulden Lomberg Chemische Fabrik GmbH**
**Byk-Gulden-Strasse 2**
**D-7750 Konstanz(DE)**

(72) Erfinder: **Amschler, Hermann, Dr.**
**Hohenhewenstrasse 19**
**D-7760 Radolfzell(DE)**
Erfinder: **Flockerzi, Dieter, Dr.**
**Ackerweg 26**
**D-7753 Allensbach(DE)**
Erfinder: **Klemm, Kurt, Prof. Dr.**
**Im Weinberg 2**
**D-7753 Allensbach(DE)**
Erfinder: **Kohl, Bernhard, Dr.**
**Heinrich-v.-Tettingen-Strasse 35a**
**D-7750 Konstanz(DE)**
Erfinder: **Ulrich, Wolf-Rüdiger, Dr.**
**Hebelstrasse 3**
**D-7750 Konstanz(DE)**

(54) **Neue Zwischenprodukte und Verfahren.**

(57) Die Erfindung betrifft enantiomer reine oder als Racemat vorliegende Verbindungen der Formel ZW

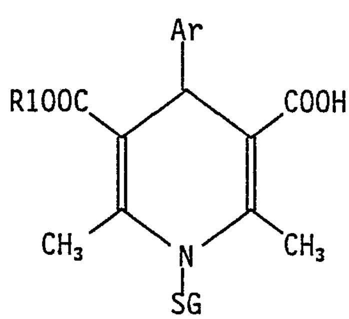

(ZW)

worin die Substituenten die in der Beschreibung angegebenen Bedeutungen haben. ein Verfahren zu ihrer Herstellung sowie ein Verfahren zur Umsetzung dieser Verbindungen.

EP 0 287 828 A1

# Neue Zwischenprodukte und Verfahren

## Anwendungsgebiet der Erfindung

Die Erfindung betrifft neue Zwischenprodukte, ein neues Verfahren zu ihrer Herstellung sowie ein neues Verfahren zur Umsetzung der Zwischenprodukte. Die aus den Zwischenprodukten erhältlichen Endprodukte werden in der pharmazeutischen Industrie zur Herstellung von Arzneimitteln eingesetzt.

## Bekannter technischer Hintergrund

Es ist bekannt, daß bestimmte, in 4-Position substituierte 1,4-Dihydropyridinderivate pharmakologisch nützliche Eigenschaften aufweisen. Ferner ist bekannt, daß diese 1,4-Dihydropyridinderivate - sofern sie in den Positionen 2 und 6 und/oder in den Positionen 3 und 5 unterschiedlich (unsymmetrisch) substituiert sind - in der Position 4 ein Chirlitätszentrum aufweisen. Außerdem ist bekannt, daß die pharmakologischen Eigenschaften der 1,4-Dihydropyridine von der absoluten Konfiguration in der 4-Position beeinflußt werden können. Aufgrund dieser unterschiedlichen pharmakologischen Eigenschaften wurde schon verschiedentlich versucht, enantiomer reine 1,4-Dihydropyridine herzustellen [siehe z.B. DE-OS 2935441, DE-OS 3320616, EP-A2 -166296, Shibaruma et al. Chem. Pharm.Bull. 28, 2809 (1980)]. Mit dem bekannten Verfahren lassen sich jedoch entweder nicht alle formelmäßig erfaßten Verbindungen herstellen (DE-OS 29 35 451) oder die beschriebenen Verfahren liefern die enantiomer reinen 1,4-Dihydropyridine nur in geringen Ausbeuten oder in einer ungenügenden Reinheit.

## Beschreibung der Erfindung

Überraschenderweise wurde nun ein neuer Weg zu enantiomer reinen, optisch aktiven 1,4-Dihydropyridinen gefunden, der die gewünschten Endprodukte in guter Ausbeute und hoher Reinheit liefert. Dieser Weg führt über teils neue, teils bekannte, als Racemat oder in anantiomer reiner Form vorliegende Zwischenprodukte der Formel ZW

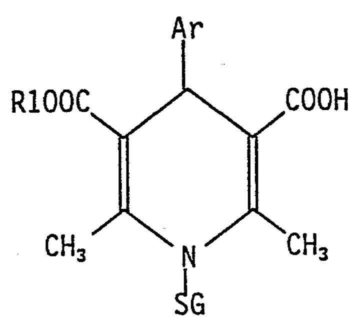

(ZW)

worin
R1 Methyl, Ethyl, Isobutyl oder Isopropyl bedeutet,
Ar einen 2-Chlorphenyl-, 3-Chlorphenyl-, 2,3-Dichlorphenyl-, 2-Nitrophenyl, 3-Nitrophenyl-, Benzoxdiazolyl-(4-Benzofurazanyl-), 2-Trifluormethylphenyl-, 2,3-Methylendioxyphenyl-oder 2,-Difluormethoxyphenylrest bedeutet und
SG eine Schtuzgruppe darstellt,
und ihre Salze.

Gegenstand der Erfindung sind somit in einem ersten Aspekt neue, enantiomer reine oder als Racemat vorliegende Verbindungen der Formel ZW, worin R1, Ar und SG die vorstehend angegebenen Bedeutungen haben, und ihre Salze mit Basen, wobei R1 nicht die Bedeutung Methyl hat wenn Ar einen 3-Nitrophenylrest

und SG einen Ethoxymethylrest darstellt.

Als Schutzgruppen SG kommen in erster Linie solche Gruppen in Frage, die in die der Verbindung ZW zugrundeliegenden Vorprodukte leicht und in hohen Ausbeuten eingeführt werden können, die bei der weiteren Umsetzung von ZW keine Nebenreaktionen eingehen und die am Ende glatt wieder abgespalten werden können. Als bevorzugte Schutzgruppen SG seien beispielsweise Alkoxymethylgruppen und Benzyloxymethylgruppen, insbesondere die Ethoxymethylgruppe genannt.

Als Salze kommen prinzipiell alle Salze mit anorganischen oder organischen Basen in Frage. Bevorzugt sind Salze mit optisch aktiven, enantiomer reinen Basen, die eine Trennung der Racemate der Zwischenprodukte ZW durch Bildung von diastereomeren Salzen ermöglicht. Als beispielhafte besonders bevorzugte Basen seien Cinchonidin oder Cinchonin genannt.

Weiterer Gegenstand der Erfindung ist ein neues Verfahren zur Herstellung der Zwischenprodukte ZW. Das Verfahren ist dadurch gekennzeichnet, daß man in Haloethylester der Formel I (Hal = Chlor, insbesondere Brom)

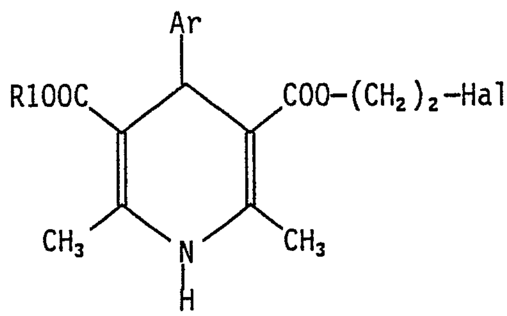

(I)

die Schutzgruppe SG einführt, in dem so erhaltenen, N-geschützten Haloethylester der Formel II

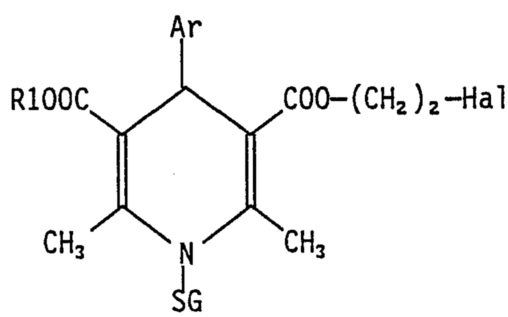

(II)

Hal gegen ein als Akzeptor ( = A) fungierendes Nucleophil austauscht, aus dem erhaltenen Akzeptorethylester der Formel III

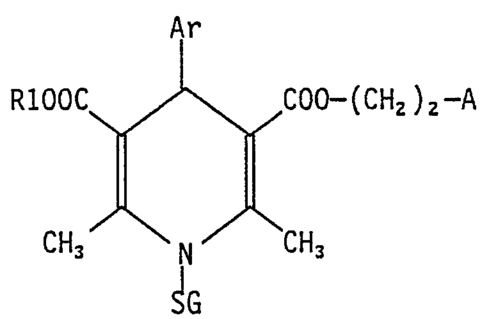

(III)

unter basischen Bedingungen den Akzeptorethylrest abspaltet und gewünschtenfalls das so erhaltene Racemat der Zwischenproduktes ZW in die Enantiomeren spaltet.

Zur Einführung der Schutzgruppe SG wird zweckmäßigerweise so vorgegangen, daß der Haloethylester I in 1-Stellung deprotoniert und anschließend mit einer Verbindung SG-X umgesetzt wird, wobei X für eine

geeignete Fluchtgruppe steht.

Als Deprotonierungsmittel kommen vor allem solche Agenzien in Frage, für die die Acidität des Protons am Stickstoff groß genug ist um eine Anionbildung zu erzielen. Neben metallorganischen Verbindungen, wie z.B. Butyllithium, seien vorzugsweise Metallhydride, insbesondere das Natriumhydrid erwähnt. Die Flucht gruppe X der Verbindung SG-X ist eine Gruppe, die bei der Umsetzung von SG-X mit dem deprotonierten I leicht abgespalten wird. Falls die Schutzgruppe SG eine Alkoxymethylgruppe ist, ist X vorzugsweise ein Halogenatom, insbesondere ein Chloratom.

Die Deprotonierung und anschließende Einführung der Schutzgruppe wird in inerten, wasserfreien Lösungsmitteln vorgenommen, wie sie für das Arbeiten mit starken Deprotonierungsmitteln geeignet sind. Beispielsweise seien offenkettige oder cyclische Ether, wie Diethylether, Dioxan oder Tetrahydrofuran genannt. Die Umsetzung erfolgt bevorzugt unter schonenden Reaktionsbedingungen bei Temperaturen um oder unter 0°C.

Besonders überraschend und für den Fachmann unerwartet ist, daß bei der vorstehend beschriebenen Deptrotonierung/Schutzgruppeneinführung die Anionbildung sauber und gezielt am Stickstoff erfolgt, obwohl aufgrund des Halogenethylrestes weitere aktivierte Protonen im Molekül vorhanden sind, und daß die Schutzgruppe glatt und in hohen Ausbeuten eingeführt werden kann, ohwohl eine Konkurrenzreaktion durch den Halogenethylrest hätte erwartet werden können.

Als Akzeptorreste bei der weiteren Umsetzung kommen insbesondere solche Reste in Frage, die einen mesomer elektronenanziehenden Effekt ausüben können. Beispielsweise seien die Nitrogruppe oder die Azidogruppe bevorzugt die Nitrilgruppe oder eine substituierte Sulfonylgruppe genannt.

Der Halogenaustausch im N-geschützten Haloethylester der Formel II erfolgt vorzugsweise durch Umsetzung mit Salzen deren Anionen geeignet sind, als kovalent gebundene Akzeptorsubstituenten zu fungieren. Bevorzugt ist die Umsetzung mit anorganischen Cyaniden, insbesondere mit Kaliumcyanid oder Natriumcyanid, oder die Umsetzung mit Alkalisulfinaten, insbesondere mit Natrium-4-toluolsulfinat, gewünschtenfalls unter Zusatz katalytischer Mengen eines quartären Ammoniumsalze, wie z.B. Tetrabutylammoniumcyanid oder Benzyltriethylammoniumchlorid, in inerten, vorzugsweise aprotischen, polaren Lösungsmitteln wie besipielsweise Aceton, Dimethylsulfoxid oder Dimethylformamid, bei Temperaturen, die bevorzugt zwischen 0°C und 50°C liegen.

Die sich an den Halogenaustausch anschließende Abspaltung des Akzeptor-ethylrestes unter basischen Bedingungen ist eine dem Fachmann geläufige Reaktion, wie sie z.B. in der DE-OS 28 47 237 beschrieben ist. Das als Racemat anfallende Zwischenprodukt (±)-ZW kann mit Hilfe enantiomer reiner, optisch aktiver Basen in üblicher Weise über die diastereomeren Salze in die Enantiomeren (+)-ZW und (-)-ZW getrennt werden [siehe z.B. Chem. Pharm. Bull. 28, 2809 (1980)] [1].

Das vorstehend beschriebene erfindungsgemäße Verfahren liefert die gewünschten Zwischenprodukte ZW ausgehend von billigen und leicht zugänglichen Ausgangsverbindung glatt und in wesentlich höheren Ausbeuten, als dies beispielsweise aus [1] bekannt ist.

Insbesondere der mit nur geringen Ausbeuten verbundene, aus [1] bekannte Schritt der Verseifung eines Methylesters wird durch das erfindungsgemäße Verfahren elegant umgangen. In dem scheinbaren Umweg Haloethylester → Akzeptorethylester → Säure, der insgesamt hohe Ausbeuten liefert, liegt der besondere erfinderische Aspekt des Verfahrens, bei dem Haloethyrest die Rolle einer reaktiven Schutzgruppe spielt, die eingeführt wird, um die (wie oben ausgeführt überraschend glatt verlaufende) Anbindung der Schutzgruppe SG vornehmen zu können, die ansonsten - ausgehend vom an sich gewünschten Akzeptorethylester - wegen dominierender Nebenreaktionen nicht gelingen würde.

Die enantiomer reinen Verbindungen der Formel ZW stellen wertvolle Zwischenprodukte zur Herstellung enantiomer reiner 1,4-Dihydropyridine dar. Diese Tatsache konnte bisher nicht bzw. zu wenig genutzt werden, da

- nach dem aus [1] bekannten Verfahren die bekannte Verbindung ZW mit Ar = 3-Nitrophenyl, R1 = Methyl und SG = Ethoxymethyl nur schwer zugänglich was und demzufolge die neuen Verbindung ZW entsprechend schwer zugänglich war erschienen, und da

- das aus [1] bekannte Verfahren zur weiteren Umsetzung von ZW nur in schlechten Ausbeuten zu einer begrenzten Zahl von 1,4-Dihydropyridinen führt.

Weiterer Gegenstand der vorliegenden Erfindung ist nunmehr ein neues Verfahren, das - ausgehend von Verbindungen der Formel ZW - zu einem breiten Spektrum von enantiomer reinen 1,4-Dihydropyridinen mit interessanten pharmakologischen Eigenschaften führt.

Das Verfahren ist dadurch gekennzeichnet, daß man enantiomer reine Verbindungen der Formel ZW mit Verbindungen der Formel IV

Y-$(CH_2)_n$-Z (IV)

worin
Y ein Halogenatom darstellt,
n die Zahl 1, 2 oder 3 bedeutet und
Z ein Wasserstoffatom ( = H) oder außerdem (wenn n = 2 oder 3 ist) Y darstellt,
umsetzt und anschließend die Schutzgruppe SG unter sauren Bedingungen abspaltet, und gegebenenfalls danach (falls Z Y darstellt) den erhaltenen Halogenalkyiester der Formel V

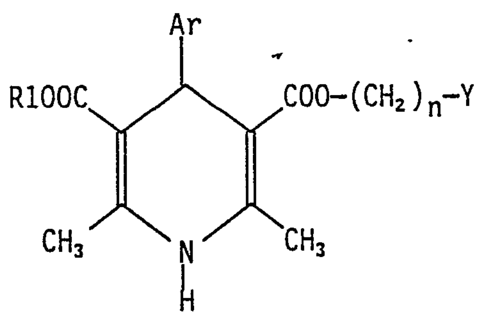

(V)

worin n 2 oder 3 bedeutet mit Aminen der Formel VI umsetzt

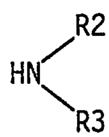

(VI)

worin
R2 Methyl und
R3 Benzyl bedeutet oder
R2 und R3 gemeinsam und mit dem Stickstoffatom, woran beide gebunden sind, einen 5-bis 7-gliedrigen Ring bilden, der als weiteres Heteroatom Sauerstoff oder Schwefel enthalten kann.

Die Umsetzung von Verbindungen ZW mit Verbindungen IV führt also nach Abspaltung der Schutzgruppe SG (je nach Art des Substituenten Z) entweder zu pharmakologisch wirksamen Endprodukten oder zu Zwischenprodukten V, die ihrerseits zu pharmakologisch wirksamen Endprodukten umgesetzt werden können.

Das Halogenatom Y ist ein Chloratom, vorzugsweise ein Brom-oder Jodatom.

Die Definition $(CH_2)_n$ in Formel IV ist als Summenformel aufzufassen. Entsprechend sind als beispielhafte bevorzugte Verbindungen der Formel IV zu nennen: Methyliodid, Ethyliodid, Isopropyliodid, 1,2-Dibromethan und 1,3-Dibrompropan.

Die Alkylierung von ZW mit IV erfolgt unter basischen Bedingungen in Gegenwart eines Phasentransferkatalysators.

Als Katalysatoren seien neben Oniumsalzen, wie z.B. Tetrabutylammoniumbromid oder Benzyltriethylammoniumchlorid, vor allem Kronenether, wie Dibenzo-[18]krone-6, Dicyclohexyl-[18]krone-6 und insbesondere [18]Krone-6 erwähnt.

Als Basen, die wenigstens im molaren Verhältnis, vorzugsweise im Überschuß eingesetzt werden, kommen anorganische Basen, wie Alkalimetallhydroxide (z.B. Natrium-oder Kaliumhydroxid), oder insbesondere Alkalimetallcarbonate (z.B. Natrium-oder vorzugsweise Kaliumcarbonat) in Frage. Beim Arbeiten in einem wassersfreien Lösungsmittel werden die verwendeten Hydroxide bzw. Carbonate vorzugsweise in feingepulverter Form eingesetzt.

Die Umsetzung erfolgt (je nach Art des Phasentransferkatalysators und der eingesetzten Base) in wasserhaltigen oder wasserfreien organischen Lösungsmitteln, oder in einem Gemisch aus Wasser und einem mit Wasser nicht oder kaum mischbaren organischen Lösungsmittel. Als Wasser/Lösungsmittelmischungen seien beispielsweise die Mischungen von Wasser mit Chloroform, Dichlormethan oder Benzol genannt. Als wasserhaltige oder wasserfreie Lösungsmittel seien beispielsweise Dichlormethan, Acetonitril oder Aceton genannt.

Die in den Beispielen genannten Lösungsmittel, Basen und Phasentransferkatalysatoren stellen nur eine exemplarische Auswahl dar. Welche weiteren Kombinationen von Lösungsmitteln, Basen und Phasentransferkatalysatoren noch geeignet sind, ist dem Fachmann aufgrund seines Fachwissens geläufig.

Die Wahl der Reaktionstemperatur bei der Umsetzung von ZW mit Verbindung IV hängt von den übrigen Reaktionsbedingungen ab, wobei in der Regel Temperaturen zwischen 20°C und der Siedetemperatur des eingesetzten Lösungsmittels bevorzugt sind.

Besonders überraschend ist in diesem Zusammenhang, daß Alkylierungsmittel wie z.B. Ethyliodid, 1,2-Dibromethan oder 1,3-Dibrompropan unter den erfindungsgemäßen Verfahrensbedingungen glatt und ohne Nebenreaktionen zu den Produkten V abreagieren, im Gegensatz zu den bisher beschriebenen Verfahren, bei denen Alkohole nach dem Säuerchloridverfahren oder in Gegenwart von Kondensationsmitteln umgesetzt werden.

Die Abspaltung der Schutzgruppe erfolgt vorzugsweise unter sauren Bedingungen. Beispielsweise kann dies - wie aus dem Stand der Technik bekannt - in verdünnter Mineralsäure geschehen. Wegen erheblicher Nebenreaktionen bei der Verwendung von Mineralsäuren wird die Abspaltung der Schutzgruppe erfindungsgemäß jedoch vorteilhafterweise mit wasserfreier Ameisensäure vorgenommen, einem Agens, das für die Abspaltung von Schutzgruppen ansonsten nicht gebräuchlich ist und das in überraschend glatter Reaktion zu seht reinen Endprodukten führt.

Wenn Z in Formel IV ein Wasserstoffatom (= H) bedeutet, so werden (ausgehend von enantiomer reinen Verbindungen ZW) unter der Bedingung, daß R1 eine von $(CH_2)_n$-H verschiedene Bedeutung hat, optisch aktive, enantiomer reine 1,4-Dihydropyridine erhalten. Es muß besonders betont werden, daß das hier vorgestellte Verfahren erstmals die Möglichkeit eröffnet, enantiomer reine 1,4-Dihydropyridine, die in den Positionen 3 und 5 von primären Alkoholen abgeleitete unterschiedliche Estergruppen tragen, in guten Ausbeuten und hoher Reinheit zu erhalten. Das aus der DE-OS 2935451 bzw. aus dem Europäischen Patent 0026317 bekannte Umesterungsverfahren ist nur bei Vorliegen einer von einem sekundären Alkohol abgeleiteten Estergruppe R100C-(z.B. bei einem Isopropylester) erfolgreich anwendbar und durch Beispiel belegt. Durch das erfindungsgemäße Verfahren werden somit erstmals die folgenden optisch aktiven anantiomer reinen 1,4-Dihydropyridine in guten Ausbeuten und in besonders reiner Form zugänglich:
(+)-1,4-Dihydro-2,6-dimethyl-4-(2,3-dichlorphenyl)-pyridin-3,5-dicarbonsäure-3-ethyl-5-methylester oder
(-)-1,4-Dihydro-2,6-dimethyl-4-(2,3-dichlorphenyl)-pyridin-3,5-dicarbonsäure-3-ethyl-5-methylester,
(+)-1,4-Dihydro-2,6-dimethyl-4-(3-nitrophenyl)-pyridin-3,5-dicarbonsäure-3-ethyl-5-methylester oder.
(-)-1,4-Dihydro-2,6-dimethyl-4-(3-nitrophenyl)-pyridin-3,5,-dicarbonsäure-3-ethyl-5-methylester,
(+)-1,4-Dihydro-2,6,-dimethyl-4-[2,3-(methylendioxy)-phenyl]-pyridin-3,5-dicarbonsäure-3-ethyl-5-methylester oder
(-)-1,4-Dihydro-2,6-dimethyl-4-[2,3-(methylendioxy)-phenyl]-pyridin-3,5-dicarbonsäure-3-ethyl-5-methylester.

Wesentlich besser und reiner als nach dem Stand der Technik bekannt werden aber beispielsweise auch folgende Verbindungen durch das erfindungsgemäße Verfahren zugänglich:
(+)-1,4-Dihydro-2,6-dimethyl-4-(4-benzofurazanyl)-pyridin-3,5-dicarbonsäure-3-isopropyl-5-methylester oder
(-)-1,4-Dihydro-2,6-dimethyl-4-(4-benzofurazanyl)-pyridin-3,5-dicarbonsäure-3-isopropyl-5-methylester,
(+)1,4-Dihydro-2,6-dimethyl-4-(2-nitrophenyl)-pyridin-3,5-dicarbonsäure-3-isopropyl-5-methylester oder
(-)-1,4-Dihydro-2,6-dimethyl-4-(2-nitrophenyl)-pyridin-3,5-dicarbonsäure-3-isopropyl-5-methylester,
(+)-1,4-Dihydro-2,6-dimethyl-4-(3-nitrophenyl)-pyridin-3,5-dicarbonsäure-3-isopropyl-5-methylester oder
(-)-1,4-Dihydro-2,6-dimethyl-4-(3-nitrophenyl)-pyridin-3,5-dicarbonsäure-3-isopropyl-5-methylester.

Die weitere Umsetzung der Zwischenprodukte V mit Aminen der Formel VI erfolgt auf eine Weise, wie sie für die Umsetzung von Alkylhalogeniden mit sekundären Aminen dem Fachmann vertraut ist.

Die Umsetzung wird in geeigneten, vorzugsweise inerten organischen Lösungsmitteln in Gegenwart von Wasser oder ohne Wasser durchgeführt. Beispielsweise seien genannt Ether, wie Dioxan, Diethylether, Tetrahydrofuran, Glykolmonoethyl ether oder Glykoldimethylether; Ketone, wie Aceton oder Ethylmethylketon; aromatische Kohlenwasserstoffe, wie Xylol oder Toluol; oder chlorierte Kohlenwasserstoffe, wie Methylenchlorid, Chloroform, Tetrachlorethylen oder Dichlorethan.

Die Reaktionstemperaturen können - je nach Reaktivität der Edukte - in einem weiten Bereich variieren. Im allgemeinen wird die Umsetzung bei Temperaturen zwischen 20°C und 150°C, vorzugsweise zwischen 20°C und 100°C, insbesondere bei der Siedetemperatur des verwendeten Lösungsmittels durchgeführt.

Das Verfahren kann bei Normaldruck oder bei erhöhtem Druck durchgeführt werden, wobei das Arbeiten bei Normaldruck die Regel ist. Die Reaktion wird in Gegenwart einer Base (z.B. eines anorganischen Carbonates, wie Kaliumcarbonat) oder unter Einsetzung eines Überschusses an Amin VI durchgeführt.

Durch das erfindungsgemäße Verfahren (Umsetzung von ZW mit einem omega-Dihalogenalkan, Abspaltung der Schutzgruppe SG und anschließende Aminierung) werden erstmals optisch aktive, enantiomer

reine 1,4-Dihydropyridine mit basischem Rest zugänglich, die bisher nicht oder nur in schlechten Ausbeuten oder in geringer Reinheit [1] erhältlich waren.

Als beispielhafte, durch das erfindungsgemäße Verfahren herstellbare Verbindungen seien besonders genannt:
(-)-1,4-Dihydro-2,6-dimethyl-4-(3-nitrophenyl)-pyridin-3,5-dicarbonsäure-3-methyl-5-[3-(benzylmethylamino)-propyl]-ester,
(-)1-,4-Dihydro-2,6-dimethyl)-4-(3-nitrophenyl)-pyridin-3,5-dicarbonsäure-3-methyl-5-[2-(benzylmethylamino)-ethyl]-ester,
(-)-1,4-Dihydro-2,6-dimethyl-4-(3-nitrophenyl)-pyridin-3,5-dicarbonsäure-3-methyl-5-[2-(4-morpholino)-ethyl]-ester,
(-)-1,4-Dihydro-2,6-dimethyl-4-(3-nitrophenyl)-pyridin-3,5-dicarbonsäure-3-methyl-5-[2-(1-piperidino)-ethyl]-ester,
(-)-1,4-Dihydro-2,6-dimethyl-4-(3-nitrophenyl)-pyridin-3,5-dicarbonsäure-3-methyl-5-[2-(1-pyrrolidino)-ethyl]-ester,
(-)-1,4-Dihydro-2,6-dimethyl-4-(3-nitrophenyl)-pyridin-3,5-dicarbonsäure-3-methyl-5-[3-(4-morpholino)-propyl]-ester,
(-)-1,4-Dihydro-2,6-dimethyl-4-(3-nitrophenyl)-pyridin-3,5-dicarbonsäure-3-methyl-5-[3-(1-piperidino)-propyl]-ester,
(-)-1,4-Dihydro-2,6-dimethyl-4-(3-nitrophenyl)-pyridin-3,5-dicarbonsäure-3-methyl-5-[3-(1-pyrrolidino)-propyl]-ester,
(+)-1,4-Dihydro-2,6-dimethyl-4-(3-nitrophenyl)-pyridin-3,5-dicarbonsäure-3-methyl-5-[3-(benzylmethylamino)-propyl]-ester,
(+)-1,4-Dihydro-2,6-dimethyl-4-(3-nitrophenyl)-pyridin-3,5-dicarbonsäure-3-methyl-5-[2-(benzylmethylamino)-ethyl]-ester,
(+)-1,4-Dihydro-2,6-dimethyl-4-(3-nitrophenyl)-pyridin-3,5-dicarbonsäure-3-methyl-5-[2-(4-morpholino)-ethyl]-ester,
(+)-1,4-Dihydro-2,6-dimethyl-4-(3-nitrophenyl)-pyridin-3,5-dicarbonsäure-3-methyl-5-[2-(1-piperidino)-ethyl]-ester,
(+)-1,4-Dihydro-2,6-dimethyl-4-(3-nitrophenyl)-pyridin-3,5-dicarbonsäure-3-methyl-5-[2-(1-pyrrolidino)-ethyl]-ester,
(+)-1,4-Dihydro-2,6-dimethyl-4-(3-nitrophenyl)-pyridin-3,5-dicarbonsäure-3-methyl-5-[3-(4-morpholino)-propyl]-ester,
(+)-1,4-Dihydro-2,6-dimethyl-4-(3-nitrophenyl)-pyridin-3,5-dicarbonsäure-3-methyl-5-[3-(1-piperidino)-propyl]-ester und
(+)-1,4-Dihydro-2,6-dimethyl-4-(3-nitrophenyl)-pyridin-3,5-dicarbonsäure-3-methyl-5-[3-(1-pyrrolidino)-propyl]-ester.

Die folgenden Herstellungsbeispiele sollen die Erfindung näher erläutern, ohne sie einzuschränken. Schmp. bedeutet Schmelzpunkt, h steht für Stunden, Kp. steht für Siedepunkt, Zers. bedeutet Zersetzung.

## Beispiele

### Endprodukte

1. (-)-1,4-Dihydro-2,6-dimethyl-4-(3-nitrophenyl)-pyridin-3,5-dicarbonsäure-3-ethyl-5-methylester

5,4 g (-)-1-Ethoxymethyl-1-,4-dihydro-5-methoxycarbonyl-2,6-dimethyl-4-(3 -nitrophenyl)-pyridin-3-carbonsäure/Cinchonidinsalz werden in 60 ml Chloroform gelöst und die Lösung unter Eiskühlung mit 40 ml 0,2N Salzsäurelösung kräftig ausgerührt, wobei durch tropfenweise Zugabe von 2N Salzsäurelösung pH2 eingestellt wird. Nach Phasentrennung wird die organische Phase noch 3 - 4 mal mit Salzsäurelösung von pH2 gewaschen bis kein Cinchonidin nehr dünnschichtchromatografisch nachzuweisen ist. Nach Neutralwaschen der organischen Phase wird über Natriumsulfat getrocknet und im Vakuum eingeengt. Das zurückbleibende Öl wird in 50 ml Aceton gelöst, dann werden 2,4 g fein gepulvertes Kaliumcarbonat, 1 ml Ethyliodid und eine Spatelspitze [18]-Krone-6 zugegeben und das Gemisch 5-6 h bei 50°C gerüht. Es wird vom Feststoff abgesaugt, das Filtrat eingeengt, der Rückstand in Dichlormethan aufgenommen, die Lösung

mit Wasser gewaschen, dann über Natriumsulfat getrocknet und wieder eingeengt. Der ölige Rückstand wird unter Kühlung mit 25 ml konzentrierter Ameisensäure versetzt und das Gemisch dann so lange gerührt, bis eine klare Lösung entstanden ist (ca. 15 - 20 Minuten). Die Ameisensäure wird im Vakuum abdestilliert, der Rückstand in Dichlormethan gelöst, die Lösung mit Natriumhydrogencarbonat-Lösung ausgeschüttelt, über Natriumsulfat getrocknet und im Vakuum wieder eingeengt. Der Rückstand wird - schließlich aus Ethanol in der Kälte kristallisiert, dann abgesaugt, mit kaltem Ethanol gewaschen und getrocknet. Man erhält 2,2 g der Titelverbindung vom Schmp. 158°C und $[\alpha]_D^{20}$ = -18,05° (c = 0,41, Ethanol).

2. (-)-4-(2,3-Dichlorphenyl)-1,4-dihydro-2,6-dimethyl-pyridin-3,5-dicarbonsäure-3-ethyl-5-methylester

2 g (+)-4-(2,3-Dichlorphenyl)-1-ethoxymethyl-1,4-dihydro-2,6-dimethyl-5-methoxycarbonyl-pyridin-3-carbonsäure werden in 30 ml Aceton gelöst und mit 1,4 g fein gepulvertem Kaliumcarbonat, 0,6 ml Ethyljodid und 50 mg [18]Krone-6 ver setzt. Das Gemisch wir 6 Stunden bei 50°C gerührt, dann wird vom Feststoff abgesaugt, das Filtrat eingeengt und der Rückstand in Dichlormethan aufgenommen. Die Lösung wird mit Wasser gewaschen, über Natriumsulfat getrocknet und wieder eingeengt. Der ölige Rückstand wird unter Kühlung mit 20 ml konzentrierter Ameisensäure versetzt; das Gemisch wird anschließend so lange gerührt, bis eine klare Lösung entstanden ist (ca. 15 - 20 Minuten). Die Ameisensäure wird im Vakuum abdestilliert, der Rückstand in Dichlormethan gelöst, die Lösung mit Natriumhydrogencarbonat-Lösung ausgeschüttelt, über Natriumsulfat getrocknet und in Vakuum wieder eingeengt. Der Rückstand wird über eine Kieselgelsäule mit Chloroform / Ethanol 95:5 als Eluens chromatographiert. Die chromatographisch reinen Fraktionen werden eingedampft und der Rückstand wird mit Diisopropylether angerieben. Es wird abgesaugt, mit kaltem Diisopropylether gewaschen und getrocknet. Man erhält 1,4 g der Titelverbindung vom Schmp. 144 -145°C und $[\alpha]_D^{22}$ = -7,2° (c=1, Methanol).

Ausgangsverbindungen

A. (+)-1,4-Dihydro-2,6-dimethyl-4-(3-nitrophenyl)-pyridin-3,5-dicarbonsäure-3-methyl-5-(3-brompropyl)-ester

35 g (-)-1-Ethoxymethyl-1,4-dihydro-5-methoxycarbonyl-2,6-dimethyl-4-(3-nitrophenyl)-pyridin-3-carbonsäure/Cinchonidinsalz werden in 250 ml Chloroform gelöst, mit 260 ml 0,2 N Salzsäurelösung versetzt und kräftig gerührt. Durch Zugabe von 2N Salzsäurelösung wird pH 2 eingestellt, dann werden die Phasen getrennt. Die organische Phase wird insgesamt viermal mit Salzsäurelösung von pH 2 und dann mit Wasser gewaschen, über Natriumsulfat getrocknet und eingeengt. Der ölige Rückstand wird in 200 ml Aceton gelöst. Anschließend werden 16 g feingepulvertes Kaliumcarbonat, 80 ml 1,5-Dibrompropan und 0,5 g [18]-Krone-6 zugegeben. Das Gemisch wird 16 h kräftig bei Raumtemperatur gerührt, dann wird abgesaugt und der Filterkuchen mit Aceton gewaschen. Das Aceton wird am Rotationsverdampfer bei - schwachem Vakuum abgezogen und das überschüssige 1,3-Dibrompropan bei 0,02 mbar abdestilliert. Der ölige Rückstand wird unter Eiskühlung mit 160 ml konzentrierter Ameisensäure übergossen; dann wird bei Raumtemperatur so lange gerührt, bis eine klare Lösung entstanden ist (ca. 15 Min.). Die Ameisensäure wird im Vakuum abdestilliert. Nach zweimaliger Zugabe und Abdestilieren von je 50 ml Toluol wird der Rückstand in Dichlormethan gelöst. Die Lösung wird mit Natriumhydrogencarbonat-Lösung ausgerührt (pH 8,5). Die organische Phase wird über Natriumsulfat getrocknet und eingeengt. Der ölige Rückstand wird dann aus Methanol in der Kälte kristallisiert. Man erhält 18,9 g der Titelverbindung vom Schmp.: 112-114°C und $[\alpha]_D^{22}$ = +13,8° (c = 1, Methanol).

B. (-)-1-Ethoxymethyl-1,4-dihydro-5-methoxycarbonyl-2,6-dimethyl-4-(3-nitrophenyl)-pyridin-3-carbonsäure/Cinchonidinsalz

229,7 g (±)-1-Ethoxymethyl-1,4-dihydro-5-methoxycarbonyl-2,6-dimethyl-4-(3-nitrophenyl)-pyridin-3-carbonsäure und 173,2 g Cinchonidin werden in 2,4 l Ethanol in der Hitze gelöst. Bei langsamer Abkühlung

kristallisiert die Titelverbindung aus und wird nach 2-tägigem Stehen bei Raumtemperatur abgesaugt und mit Ethanol gewaschen. Nach zweimaligem Umkristallisieren aus Ethanol erhält man 148 g der Titelverbindung vom Schmp. 185-185,5°C und $[\alpha]_D^{22}$ = -63,4° (c = 1, Chloroform). Durch Aufarbeiten der Mutterlauge aus der 1. Kristallisation kann das entsprechende (+)-Enantiomere erhalten werden.

C. (±)-1-Ethoxymethyl-1,4-dihydro-5-methoxycarbonyl-2,6-dimethyl-4-(3-nitrophenyl)-pyridin-3-carbonsäure

a) 195,5 g (±)-1-Ethoxymethyl-1,4-dihydro-2,6-dimethyl-4-(3-nitrophenyl)-pyridin-3,5-dicarbonsäure-3-methyl-5-(2-bromethyl)-ester werden in 900 ml Dimethylformamid gelöst und mit 40,6 g gepulvertem Natriumcyanid und 0,5 g Tetrabutylammoniumcyanid versetzt. Das Gemisch wird 26 h bei Raumtemperatur gerührt, wobei nach ca. 4 h ein dicker Niederschlag ausfällt, dann werden 200 ml 2N Natriumhydroxid-Lösung zugetropft und 5 h bei Raumtemperatur weitergerührt. Das Gemisch wird in 4,5 l Wasser eingerührt und unter Kühlung und starkem Rühren mit 2N Salzsäurelösung bis pH 2,5 tropfenweise versetzt. Das dabei ausgefallene Produkt wird scharf abgesaugt, mit Wasser neutral gewaschen, über Nacht luftgetrocknet und dann in 400 ml Ethanol auf dem Dampfbad erhitzt (keine Lösung). Es wird 12 h bei Raumtemperatur stehengelassen, dann gekühlt, abgesaugt und getrocknet. Man erhält 142 g der Titelverbindung vom Schmp. 176 -176,5°C (Zers.).

b) 5 g (±)-1-Ethoxymethyl-1,4-dihydro-2,6-dimethyl-4-(3-nitrophenyl)-pyridin-3,5-dicarbonsäure-3-methyl-5-(2-bromethyl)-ester werden in 35 ml Dimethylformamid gelöst, 3,55 g (20 mMol) 4-Toluolsulfinsäure-Natrium-Salz und 0,1 g Benzyltriethylammoniumchlorid zugesetzt und 20 h bei 20°C gerührt. Man gibt 11 ml 2N Natronlauge zu, rührt 5 h bei 20°C, weitere 3 h bei 80°C und gießt nach Abkühlung auf 120 ml Eiswasser. Nach Zugabe von Essigsäure bis pH 3,8 wird 3 h im Eisbad gerührt, vom ausgefallenen Feststoff filtriert, mit Wasser neutral gewaschen und getrocknet. Das Rohprodukt wird in 10 ml Ethanol ausgekocht, abgekühlt, filtriert und getrocknet. Man erhält 3,31 g der Titelverbindung vom Schmp. 176 - 176,5°C (Zers.).

D. (±)-1-Ethoxymethyl-1,4-dihydro-2,6-dimethyl-4-(3-nitrophenyl)-pyridin-3,5-dicarbonsäure-3-methyl-5-(2-bromethyl)-ester

10,65 g einer 80 %igen Suspension von Natriumhydrid in Paraffinöl werden in 300 ml wasserfreiem Tetrahydrofuran aufgeschlämmt. Bei -5 bis -7°C wird unter Rühren eine Lösung von 120 g (±)-1,4-Dihydro-2,6-dimethyl-4-(3-nitrophenyl)-pyridin-3,5-dicarbonsäure-3-methyl-5-(2-bromethyl)-ester und 28,4 g Chlormethylethylether in 300 ml wasserfreiem Tetrahydrofuran langsam innerhalb von 4 h zugetropft. Nach einer weiteren Stunde Rühren bei 0°C werden nacheinander 600 ml Toluol und 210 ml Wasser zugegeben. Die Phasen werden getrennt, die organische Phase wird mit Wasser gewaschen, über Nacht getrocknet und im Vakuum eingeengt. Der Rückstand wird in 390 ml warmem Ethanol gelöst, dann wird unter Rühren abgekühlt. Dabei kristallisiert das Produkt aus. Es wird auf 0°C gekühlt, abgesaugt, mit eiskaltem Ethanol gewaschen und getrocknet. Man erhält 98,5 g der Titelverbindung vom Schmp. 72,5 - 74°C.

E. (±)-1,4-Dihydro-2,6-dimethyl-4-(3-nitrophenyl)-pyridin-3,5-dicarbonsäure-3-methylester-5-(2-bromethyl)-ester

494 g 2-(3-Nitrobenzyliden)-acetessigsäure-(2-bromethyl)-ester und 173,5 g 3-Aminocrotonsäuremethylester werden unter Rühren und unter einer Stickstoffatmosphäre in 1750 ml Isopropanol bei 75-80°C gelöst. Das Gemisch wird danach sehr langsam im Heizbad abgekühlt. Nach Animpfen mit der Titelverbindung wird 16 h bei Raumtemperatur gerührt, dann gekühlt, abgesaugt, mit eiskaltem Ether gewaschen und getrocknet. Man erhält 481 g der Titelverbindung vom Schmp. 139 - 141°C.

F. 2-(3-Nitrobenzyliden)-acetessigsäure-(2-bromethyl)-ester

405 g Acetessigsäure-2-bromethylester werden in 1500 ml Isopropanol gelöst. Unter Rühren werden 293 g 3-Nitrobenzaldehyd, 4,6 g Essigsäure und 6,8 g Piperidin zugegeben. Das Gemisch wird bei 40°C so lange gerührt bis eine klare Lösung entstanden ist. Man läßt langsam abkülen, impft mit einigen Kristallen der Titelverbindung an und rührt bei Raumtemperatur 48 h lang, dann wird gekühlt, abgesaugt, mit kaltem Isopropanol gewaschen und im Vakuum bei 50°C getrocknet. Man erhält 495 g der Titelverbindung vom Schmp. 94,5 - 95,5°C.

G. Acetessigsäure-2-bromethylester

250 g 2-Bromethanol werden in 1,3 l Dichlormethan gelöst und mit 1,2 g 4-Dimethylaminopyridin versetzt. Unter kräftigem Rühren werden 400 ml einer 50%igen Diketenlösung in Aceton so zugetropft, daß das Lösungsmittel mäßig siedet. Nach dem Zutropfen wird noch 1 h bei Siedetemperatur gerührt und dann über Nacht stehengelassen. Nach Abdampfen des Lösungsmittels am Rotationsverdampfer im Vakuum wird der Rückstand destilliert. Man erhält 402 g der Titelverbindung mit Kp. 80 - 83°C/0,04 mbar.

H. (+)-4-(2,3-Dichlorphenyl)-1-ethoxymethyl-1,4-dihydro-2,6-dimethyl-5-methoxycarbonyl-pyridin-3-carbonsäure

9,5 g (±)-4-(2,3-Dichlorphenyl)-1-ethoxy-methyl-1,4-dihydro-2,6-dimethyl-5-methoxycarbonyl-pyridin-3-carbonsäure und 6,75 g Cinchonidin werden in 50 ml Methanol in der Siedehitze gelöst; die heiße Lösung wird filtriert und dann abgekühlt. Nach Animpfen wird mehrere Tage bei 0 - 4°C stehengelassen, dann wird abgesaugt, mit kaltem Methanol gewaschen und nochmals aus Methanol umkristallisiert. Man erhält 4,85 g des Cinchonidinsalzes der Titelverbindung vom Schmp. 176 - 178°C. Das Salz wird in 60 ml Chloroform gelöst. Nach Zugabe von 30 ml Wasser wird unter kräftigem Rühren durch Zugabe von 2N Salzsäurelösung in der wäßrigen Phase ein pH von 2 eingestellt. Nache Phasentrennung wird die organische Phase noch 4 mal mit 0,01N Salzsäure und 1 mal mit Wasser gewaschen, über Natriumsulfat getrocknet und dann im Vakuum eingedampft. Der zurückbleibende kristalline Rückstand wird im Hochvakuum getrocknet. Man erhält 3,1 g der Titelverbindung vom Schmp. 129 - 131°C und $[\alpha]_D^{22} = +63,2°$ (c = 1, Chloroform).

I. (±)-4-(2,3-Dichlorphenyl)-1-ethoxymethyl-1,4-dihydro-2,6-dimethyl-5-methoxycarbonyl-pyridin-3-carbonsäure

Analog Beispiel C werden aus 14,25 g 4-(2,3-Dichlorphenyl)-1-ethoxymethyl-1,4-dihydro-2,6-dimethylpyridin-3,5-dicarbonsäure-3-(2-bromethyl)-5-methylester, 2,8 g gepulvertem Natriumcyanid und einer Spatelspitze Tetrabutylammoniumcyanid in 60 ml Dimethylformamid nach Zugabe von 30 ml 2N Natronlauge und identischer Aufarbeitung 9,5 g der Titelverbindung vom Schmp. 156 - 157°C (aus Ethanol) erhalten.

J. (±)-4-(2,3-Dichlorphenyl)-1-ethoxymethyl-1,4-dihydro-2,6-dimethyl-pyridin-3,5-dicarbonsäure-3-(2-bromethyl)-5-methylester

Analog Beispiel D werden aus 19,1 g (±)-4-(2,3-Dichlorphenyl)-1,4-dihydro-2,6-dimethyl-pyridin-3,5-dicarbonsäure-3-(2-bromethyl)-5-methylester, 1,64 g Natriumhydrid (80 % in Paraffinöl), 4,75 g Chlormethyl-ethylether und 2 mal 50 ml wasserfreiem Tetrahydrofuran 14,3 g der Titelverbindung vom Schmp. 104 - 106°C (aus Methanol) erhalten.

K. (±)-4-(2,3-Dichlorphenyl)-1,4-dihydro-2,6-dimethyl-pyridin-3,5-dicarbonsäure-3-(2-bromethyl)-5-methylester

50 g 2,3-Dichlorbenzaldehyd und 60 g Acetessigsäure-2-bromethylester werden in 400 ml Dichlormethan gelöst. Die Lösung wird mit 0,83 ml Eisessig, 1,4 ml Piperidin und 30 g wasserfreiem Natriumsulfat versetzt. Das Gemisch wird 4 Tage bei Raumtemperatur gerührt, wobei nach 2 Tagen nochmals 20 g wasserfreies Natriumsulfat zugesetzt werden. Nach dem Absaugen wird die Lösung mit Wasser, 0,1N Salzsäure, verdünnter Natriumhydrogencarbonat-Lösung und wieder Wasser gewaschen und über Natriumsulfat getrocknet. Nach dem Abdestillieren des Lösungsmittels im Vakuum wird der ölige Rückstand in 600 ml Tetrahydrofuran gelöst und die Lösung mit 33 g 3-Aminocrotonsäuremethylester versetzt. Das Gemisch wird 24 Stunden unter Stickstoff zum Sieden erhitzt, nach dem Abkühlen wird das Lösungsmittel im Vakuum abdestilliert, der Rückstand in Dichlormethan gelöst und die Lösung mit 0,01N Salzsäure und Wasser gewaschen. Nach Trocknen über Natriumsulfat wird wieder eingeengt. Der teilkristalline Rückstand wird mit 150 ml 2-Propanol versetzt, gekühlt und abgesaugt. Nach Umkristallisation aus 2-Propanol erhält man 79 g der Titelverbindung vom Schmp. 180 - 182°C.

**Ansprüche**

1. Enantiomer reine oder als Racemat vorliegende Verbindungen der Formel ZW

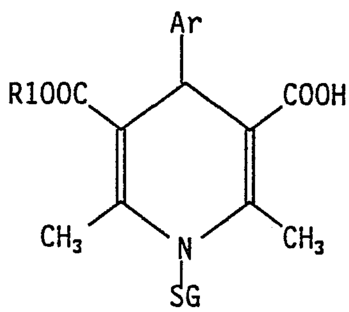

(ZW)

worin
R1 Methyl, Ethyl, Isobutyl oder Isopropyl bedeutet,
Ar einen 2-Chlorphenyl-, 3-Chlorphenyl-, 2,3-Dichlorphenyl-, 2-Nitrophenyl, 3-Nitrophenyl-, Benzoxdiazolyl-(4-Benzofurazanyl-), 2-Trifluormethylphenyl-, 2,3-Methylendioxyphenyl-oder 2-Difluormethoxyphenylrest bedeutet und
SG eine Schutzgruppe darstellt,
und ihre Salze, wobei R1 nicht die Bedeutung Methyl hat wenn Ar einen 3-Nitrophenylrest und SG einen Ethoxymethylrest darstellt.

2. Verfahren zur Herstellung der Verbindungen der Formel ZW nach Anspruch 1, worin
R1 Methyl, Ethyl, Isobutyl oder Isopropyl bedeutet,
Ar einen 2-Chlorphenyl-, 3-Chlorphenyl-, 2,3-Dichlorphenyl-, 2-Nitrophenyl, 3-Nitrophenyl-, Benzoxdiazolyl-(4-Benzofurazanyl-), 2-Trifluormethylphenyl-, 2,3-Methylendioxyphenyl-oder 2-Difluormethoxyphenylrest bedeutet und
SG eine Schutzgruppe darstellt, und ihrer Salze,
dadurch gekennzeichnet, daß man in Haloethylester der Formel I

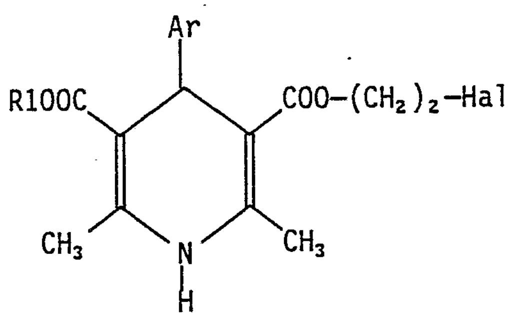

(I)

die Schutzgruppe SG einführt, in dem so erhaltenen, N-geschützen Haloethylester der Formel II

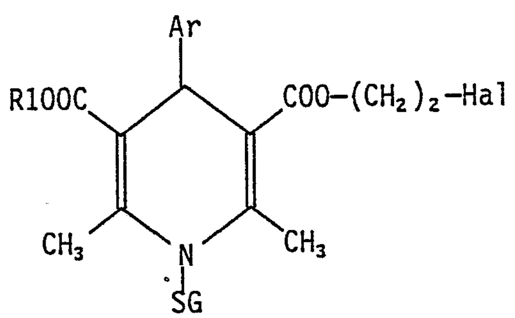

(II)

Hal gegen ein als Akzeptor (= A) fungierendes Nucleophil austauscht, aus dem erhaltenen Akzeptorethylester der Formel III

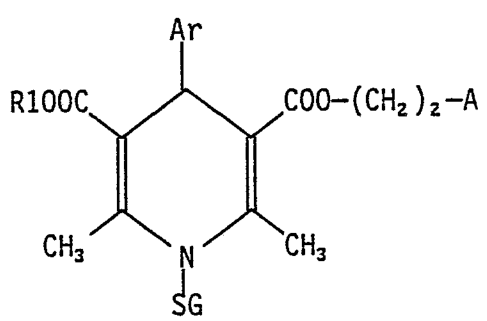

(III)

unter basischen Bedingungen den Akzeptorethylrest abspaltet und gewünschtenfalls das so erhaltene Racemat der Verbindung ZW in die Enantiomeren spaltet und/oder gewünschtenfalls anschließend in die Salze überführt.

3. Verfahren nach Anspruch 2, dadurch gekennzeichnet, daß Hal ein Bromatom ist.

4. Verfahren nach Anspruch 2, dadurch gekennzeichnet, daß die Schutzgruppe SG eine Alkoxymethyl- oder Benzyloxymethylgruppe ist.

5. Verfahren nach Anspruch 2, dadurch gekennzeichnet, daß der Akzeptor eine Cyangruppe oder eine substituierte Sulfonylgruppe ist.

6. Verfahren zur weiteren Umsetzung der Verbindungen der Formel ZW nach Anspruch 1, worin
R1 Methyl, Ethyle, Isobutyl oder Isopropyl bedeutet,
Ar einen 2-Chlorphenyl-, 3-Chlorphenyl-, 2,3-Dichlorphenyl-, 2-Nitrophenyl, 3-Nitrophenyl-, Benzoxdiazolyl-(4-Benzofurazanyl-), 2-Trifluormethylphènyl-, 2,3-Methylendioxyphenol-oder 2-Difluormethoxyphenylrest bedeutet und
SG eine Schutzgruppe darstellt,
dadurch gekennzeichnet, daß man enantiomer reine Verbindungen der Formle ZW mit Verbindungen der Formel IV

$Y\text{-}(CH_2)_n\text{-}Z$ (IV)

worin
Y ein Halogenatom darstellt,
n die Zahl 1, 2 oder 3 bedeutet und
Z ein Wasserstoffatom (= H) oder außerdem (wenn n = 2 oder 3 ist) Y darstellt,
umsetzt und anschließend die Schutzgruppe SG unter sauren Bedingungen abspaltet, und gegebenenfalls danach (falls Z Y darstellt) den erhaltenen Halogenalkylester der Formel V

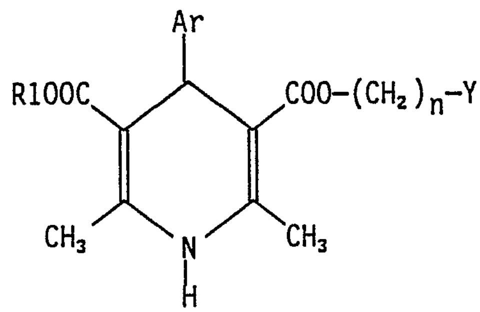
(V)

worin n 2 oder 3 bedeutet mit Aminen der Formel VI umsetzt

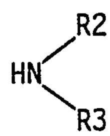
(VI) .

worin
R2 Methyl und
R3 Benzyl bedeutet oder
R2 und R3 gemeinsam und mit dem Stickstoffatom, woran beide gebunden sind, einen 5-bis 7-gliedrigen Ring bilden, der als weiteres Heteroatom Sauerstoff oder Schwefel enthalten kann.

7. Verfahren zur weiteren Umsetzung der Verbindungen der Formel ZW nach Anspruch 1, worin
R1 Methyl, Ethyl, Isobutyl oder Isopropyl bedeutet,
Ar einen 2-Chlorphenyl-, 3-Chlorphenyl-, 2,3-Dichlorphenyl-, 2-Nitrophenyl, 3-Nitrophenyl-, Benzoxidiazolyl-(4-Benzofurazanyl-), 2-Trifluormethylphenyl-, 2,3-Methylendioxyphenyl-oder 2-Difluormethoxyphenylrest bedeutet und
SG eine Schutzgruppe darstellt,
dadurch gekennzeichnet, daß man enantiomer reine Verbindungen der Formel ZW mit Verbindungen der Formel IV

$Y\text{-}(CH_2)_n\text{-}Z$ (IV)

worin
Y ein Halogenatom darstellt,
n die Zahl 1, 2 oder 3 bedeutet und
Z ein Wasserstoffatom (= H) darstellt,
umsetzt und anschließend die Schutzgruppe SG unter sauren Bedingungen abspaltet.

8. Verfahren zur weiteren Umsetzung der Verbindungen der Formel ZW nach Anspruch 1, worin
R1 Methyl, Ethyl, Isobutyl oder Isopropyl bedeutet,
Ar einen 2-Chlorphenyl-, 3-Chlorphenyl-, 2,3-Dichlorphenyl-, 2-Nitrophenyl, 3-Nitrophenyl-, Benzoxdiazolyl-(4-Benzofurazanyl-), 2-Trifluormethylphenyl-, 2,3-Methylendioxyphenyl-oder 2-Difluormethoxyphenylrest bedeutet und
SG eine Schutzgruppe darstellt,
dadurch gekennzeichnet, daß man enantiomer reine Verbindungen der Formel ZW mit Verbindungen der Formel IV

$Y\text{-}(CH_2)_n\text{-}Z$ (IV)

worin
Y ein Halogenatom darstellt,
n die Zahl 2 oder 3 bedeutet und
Z Y darstellt,
umsetzt, anschließend die Schutzgruppe SG unter sauren Bedingungen abspaltet, und danach den erhaltenen Halogenalkylester der Formel V

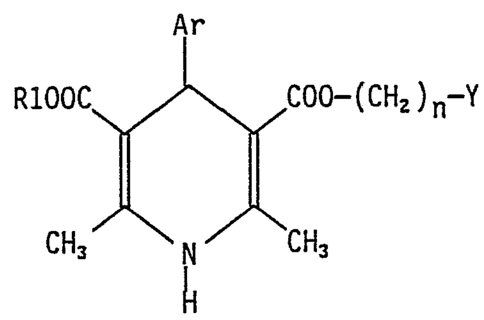

(V)

mit Aminen der Formel VI umsetzt

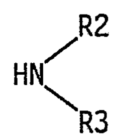

(VI)

worin
R2 Methyl und
R3 Benzyl bedeutet oder
R2 und R3 gemeinsam und mit dem Stickstoffatom, woran beide gebunden sind, einen 5-bis 7-gliedrigen Ring bilden, der als weiteres Heteroatom Sauerstoff oder Schwefel enthalten kann.

9. Verfahren nach Anspruch 6 oder 7 oder 8, dadurch gekennzeichnet, daß die Schutzgruppe SG mit wasserfreier Ameisensäure abgespalten wird.

10. Verfahren nach Anspruch 6, dadurch gekennzeichnet, daß nach diesem Verfahren eine Verbindung ausgewählt aus der Gruppe
(+)-1,4-Dihydro-2,6-dimethyl-4-(2,3-dichlorphenyl)-pyridin-3,5-dicarbonsäure-3-ethyl-5-methylester,
(-)-1,4-Dihydro-2,6-dimethyl-4-(2,3-dichlorphenyl)-pyridin-3,5-dicarbonsäure-3-ethyl-5-methylester,
(+)-1,4-Dihydro-2,6-dimethyl-4-(3-nitrophenyl)-pyridin-3,5-dicarbonsäure-3-ethyl-5-methylester oder
(-)-1,4-Dihydro-2,6-dimethyl-4-(3-nitrophenyl)-pyridin-3,5-dicarbonsäure-3-ethyl-5-methylester,
(+)-1,4-Dihydro-2,6-dimethyl-4-[2,3-(methylendioxy)-phenyl]-pyridin-3,5-dicarbonsäure-3-ethyl-5-methylester,
(-)-1,4-Dihydro-2,6-dimethyl-4-[2,3-(methylendioxy)-phenyl]-pyridin-3,5-dicarbonsäure-3-ethyl-5-methylester,
(+)-1,4-Dihydro-2,6-dimethyl-4-(4-benzofurazanyl)-pyridin-3,5-dicarbonsäure-3-isopropyl-5-methylester,
(-)-1,4-Dihydro-2,6-dimethyl-4-(4-benzofurazanyl)-pyridin-3,5-dicarbonsäure-3-isopropyl-5-methylester,
(+)-1,4-Dihydro-2,6-dimethyl-4-(2-nitrophenyl)-pyridin-3,5-dicarbonsäure-3-isopropyl-5-methylester,
(-)-1,4-Dihydro-2,6-dimethyl-4-(2-nitrophenyl)-pyridin-3,5-dicarbonsäure-3-isopropyl-5-methylester,
(-)-1,4-Dihydro-2,6-dimethyl-4-(3-nitrophenyl)-pyridin-3,5-dicarbonsäure-3-isopropyl-5-methylester,
(+)-1,4-Dihydro-2,6-dimethyl-4-(3-nitrophenyl)-pyridin-3,5-dicarbonsäure-3-methyl-5-[3-(benzylmethylamino)-propyl]-ester,
(+)-1,4-Dihydro-2,6-dimethyl-4-(3-nitrophenyl)-pyridin-3,5-dicarbonsäure-3-methyl-5-[2-(benzylmethylamino)-ethyl]-ester,
(+)-1,4-Dihydro-2,6-dimethyl-4-(3-nitrophenyl)-pyridin-3,5-dicarbonsäure-3-methyl-5-[2-(4-morpholino)-ethyl]-ester,
(+)-1,4-Dihydro-2,6-dimethyl-4-(3-nitrophenyl)-pyridin-3,5-dicarbonsäure-3-methyl-5-[2-(1-piperidino)-ethyl]-ester,
(+)-1,4-Dihydro-2,6-dimethyl-4-(3-nitrophenyl)-pyridin-3,5-dicarbonsäure-3-methyl-5-[2-(1-pyrrolidino)-ethyl]-ester,

(+)-1,4-Dihydro-2,6-dimethyl-4-(3-nitrophenyl)-pyridin-3,5-dicarbonsäure-3-methyl-5-[3-(4-morpholino)-propyl]-ester,
(+)-1,4-Dihydro-2,6-dimethyl-4-(3-nitrophenyl)-pyridin-3,5-dicarbonsäure-3-methyl-5-[3-(1-piperidino)-propyl]-ester,
(+)-1,4-Dihydro-2,6-dimethyl-4-(3-nitrophenyl)-pyridin-3,5-dicarbonsäure-3-methyl-5-[3-(1-pyrrolidino)-propyl]-ester
(-)-1,4-Dihydro-2,6-dimethyl-4-(3-nitrophenyl)-pyridin-3,5-dicarbonsäure-3-methyl-5-[3-(benzylmethylamino)-propyl]-ester,
(-)-1,4-Dihydro-2,6-dimethyl-4-(3-nitrophenyl)-pyridin-3,5-dicarbonsäure-3-methyl-5-[2-(benzylmethylamino)-ethyl]-ester,
(-)-1,4-Dihydro-2,6-dimethyl-4-(3-nitrophenyl)-pyridin-3,5-dicarbonsäure-3-methyl-5-[2-(4-morpholino)-ethyl]-ester,
(-)-1,4-Dihydro-2,6-dimethyl-4-(3-nitrophenyl)-pyridin-3,5-dicarbonsäure-3-methyl-5-[2-(1-piperidino)-ethyl]-ester,
(-)-1,4-Dihydro-2,6-dimethyl-4-(3-nitrophenyl)-pyridin-3,5-dicarbonsäure-3methyl-5-[2-(1-pyrrolidino)-ethyl]-ester,
(-)-1,4-Dihydro-2,6-dimethyl-4-(3-nitrophenyl)-pyridin-3,5-dicarbonsäure-3-methyl-5-[3-(4-morpholino)-propyl]-ester,
(-)-1,4-Dihydro-2,6-dimethyl-4-(3-nitrophenyl)-pyridin-3,5-dicarbonsäure-3-methyl-5-[3-(1-piperdino)-propyl]-ester und
(-)-1,4-Dihydro-2,6-dimethyl-4-(3-nitrophenyl)-pyridin-3,5-dicarbonsäure-3-methyl-5-[3-(1-pyrrolidino)-propyl]-ester
hergestellt wird.

Europäisches Patentamt

# EUROPÄISCHER RECHERCHENBERICHT

Nummer der Anmeldung

EP 88 10 4690

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 026 317 (BAYER AG)<br>* Seiten 4-6 * | 2-10 | C 07 D 211/90<br>C 07 D 413/04<br>C 07 D 405/04 |
| D,Y | CHEM. PHARM. BULL., Band 28, Nr. 9, 1980, Seiten 2809-2812; T. SHIBANUMA et al.: "Synthesis of optically active 2-(N-benzyl-N-methylamino)ethyl methyl 2,6-dimethyl-4-(m-nitrophenyl)-1,4-dihydropyridine-3,5-dicarboxylate (nicardipine)"<br>* Seite 2809 * | 1-10 | |
| D,A | FR-A-2 528 431 (SANDOZ S.A.)<br>* Ansprüche * | 1-10 | |
| D,A | EP-A-0 011 706 (BAYER AG)<br>* Seiten 4-7 * | 2-10 | |
| D,A | EP-A-0 166 296 (BAYER AG)<br>* Seiten 4-6 * | 2-10 | |

| RECHERCHIERTE SACHGEBIETE (Int. Cl.4) |
|---|
| C 07 D 211/00<br>C 07 D 413/00<br>C 07 D 405/00 |

Der vorliegende Recherchenbericht wurde für alle Patentansprüche erstellt

| Recherchenort | Abschlußdatum der Recherche | Prüfer |
|---|---|---|
| DEN HAAG | 20-07-1988 | BRIGHENTI |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)